# EUROPEAN PATENT SPECIFICATION

(11) **EP 3 877 040 B1**
(45) Date of publication and mention of the grant of the patent: **07.12.2022**
(21) Application number: 18836920.1
(22) Date of filing: 08.11.2018
(51) Int. Cl.: A61N 1/32

(54) **ELECTRIC DEVICE FOR THE SKIN ALLERGY TEST**
ELEKTRISCHE VORRICHTUNG FÜR DEN HAUTALLERGIETEST
APPAREIL ÉLECTRIQUE POUR LE TEST D'ALLERGIE CUTANÉE

(43) Date of publication of application: 15.09.2021
(73) Proprietor: Mediform Italia S.r.l., 95024 Acireale (CT) (IT)
(72) Inventor: MANCINI, Mario, 95024 Acireale (CT) (IT)
(86) International application number: PCT/IT2018/000145
(87) International publication number: WO 2020/095332

(56) References cited:
- EP-A2- 1 281 367
- DE-U1- 29 711 006
- FR-A1- 2 513 060
- US-A- 4 819 657
- US-A- 5 820 562
- US-A1- 2009 318 846
- US-A1- 2011 270 122
- US-A1- 2014 276 196
- US-A1- 2017 347 938

## Description

The present invention regards an electric medical device, used for the skin allergy test, to be placed in touch with a skin, preferably a forearm skin belonging to a person. It is able to print a series of areas by ink marks, and to emit on command an electric pulse, that generates a respective small surface hole on the same skin.

The device comprises means providing on command one or more allergens, by drops or by liquid micro-film, to said areas printed by ink marks, so that the allergens could go in touch with the skin surface layers, and would be possible to observe the occurring of a possible allergy reaction.

As known, in the state of the art there is the so called *prick test,* that represents a skin allergy test, and it is very important for the diagnosis of the respiratory diseases, nutritional diseases, and diseases caused by other allergens. The *prick test* is an allergy test used in order to understand when a specific substance causes an allergic inflammation, by immediate mechanism or by an IgE (Immunoglobulin E) mechanism, that is an *immediate-type hypersensitivity* or a reaction of the first type, according to the Coombs and Gell classification.

In the *prick test,* some drops of purified allergen are placed on the skin surface, usually on the forearm, and then the skin is scratched by a single-use lancet, in order to make the allergen to penetrate under the skin surface layers. After waiting approximately 15-20 minutes, the skin reaction is observed, with reference to each specific allergen that has been applied. The reaction to each single allergen is compared with a positive check (usually by using *histamine*) and with a negative check (usually by using *glycerin,* that is the standard dilution liquid for the allergens).

In case of a positive reaction, it is possible to observe a so called *hive,* that is a skin edema, similar to a mosquito bite, that is very itchy and has a perimetric erythema ring.

However, this simple test is characterized by a drawback connected to the standardization of the procedure, because it is quite impossible to repeat the same depth in penetration by different lancets. In fact, it is quite impossible to repeat exactly the same pressure and location of the lancet on the skin.

Another drawback is related to the location of drops after each application at one point, with a consequent diffusion and contamination of the adjacent application sites. Furthermore, this type of technique leads to an effect, so called *belonephobia* (otherwise known as *trypanophobia*)*,* that could cause even the state of unconsciousness in a patient affected by panic.

All the above drawbacks are overcome by the present invention, that discloses a medical device of new generation, that guarantees a perfect standardization in the procedure of the skin allergy test, due to the emission of an electric pulse having low voltage and high amperage (ion-carbonation) - technical reference: battery *850 mAh 3.7 V*, input current *5V* / *1A,* max output power *5W.*

Furthermore, the same device permits to decrease the execution time of the procedure, without pain or bleeding for the patient.

Document EP1281367 A2 discloses a laser assisted local permeation of pharmaceuticals through skin - such as anaesthetics, antibiotics, hormones, allergens, chemotherapy drugs, vaccines or DNA products. Document US2009/318846 A1 discloses a method for increasing the permeability of a barrier comprises: applying ablation material; providing a thermal treatment device; activating microheater component; heating ablation material; and increasing the permeability of a barrier.

Therefore, the main objective of the present invention to propose an electric device for the skin allergy test, to be placed in touch with a skin, preferably a forearm skin belonging to a person; said device being able to print a series of areas by ink marks, and to emit on command an electric pulse, that generates a respective small surface hole on the same skin.

Another objective is that said device comprises means providing on command one or more allergens, by drops - e.g. by a pipette or a brush dispenser, to said areas printed by ink marks, so that the allergens could go in touch with the skin surface layers, and would be possible to observe the occurring of a possible allergy reaction.

A further objective is that intensity and duration of the electric pulse emitted by the device could be defined by: tuning means based on a manual command; or tuning means based on an automatic command (skin *auto-checking*); or means that are set to factory default values.

A further objective is that the device comprises an add-on element, in a set back or front position, and that said add-on element comprises a set of elements placed on a platform.

And another further objective is that the same device comprises, at the external surface, a series of buttons for activation and/or selection of the pulse duration and intensity, and it further comprises some LED elements, having green or red color, that indicate different states of working in the same device.

Therefore, it is specific subject of the present invention an electric device for the skin allergy test, according to claim 1.

The present invention is now being described according to non-limiting examples, with particular reference to the figures of the enclosed drawings, where:
**Figure 1** is a perspective view where an allergen drop is placed on the surface skin belonging to a person;
**Figure 2** is a perspective view where a single-use lancet scratches the same skin of **Figure 1****,** so that the allergen liquid can penetrate into the skin surface;
**Figure 3** is a perspective view where the same skin of **Figure 1** has a positive reaction to the same allergen, and a skin edema shows up, called *hive,* having a perimetric erythema ring;
**Figure 4** is a front perspective view of the forearm belonging to a person, where a series of areas have been ink marked, and on each of them an allergy test has been done;
**Figure 5** is a front perspective view of an allergy test device, according to the present invention, in an assembled configuration;
**Figure 6** is a front perspective view of the same allergy test device of **Figure 5****,** in a disassembled configuration;
**Figure 7** is a front perspective view of components belonging to the same allergy test device, in a disassembled configuration, which are a nozzle and an ink tank;
**Figure 8** is a front perspective view of a prototype representing the same allergy test device;
**Figure 9** is a front schematic perspective view of the same allergy test device applied to a skin surface;
**Figures 10-12** are respective front views of a prototype representing the same allergy test device, in a sequence of steps applying to a skin surface;
**Figures 13, 15** and **17** are respective front, lateral and top views, of an add-on element in a set back position, applied to the same allergy test device;
**Figures 14, 16 and 18** are respective front, lateral and top views, of an add-on element in a set front position, applied to the same allergy test device;
**Figure 19** is a view of a block diagram related to means of generation, transmission and control of an electric pulse, belonging to the same allergy test device.

It is underlined that only few of the many conceivable embodiments of the present invention are here described, which are just some specific non-limiting examples, having the possibility to describe many other embodiments based on the disclosed technical solutions of the present invention.

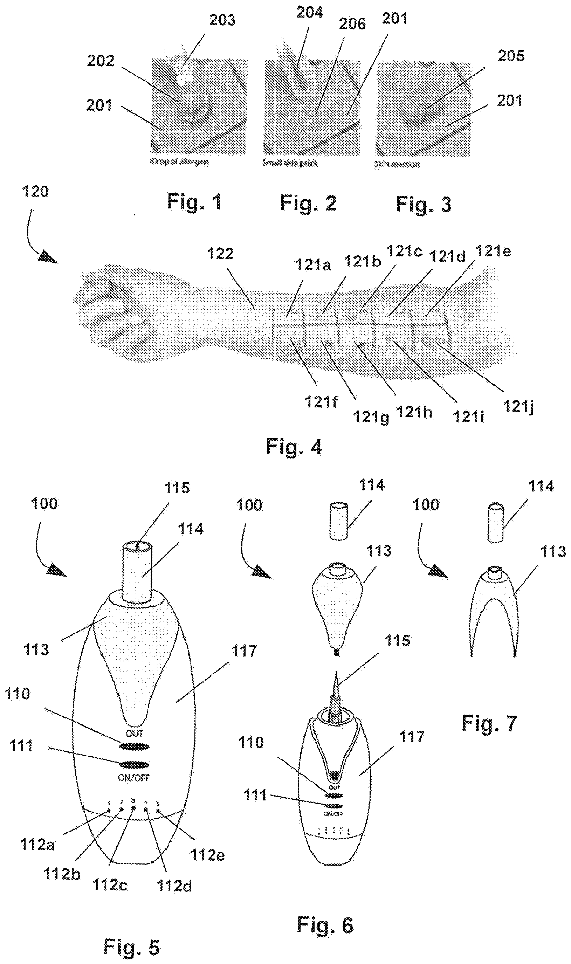

**Figures 5, 6, 7** **and** **8** show an electric device **100** for the skin allergy test, essentially comprising: a main body **117,** where a metallic tip **115** is installed on the top of it.

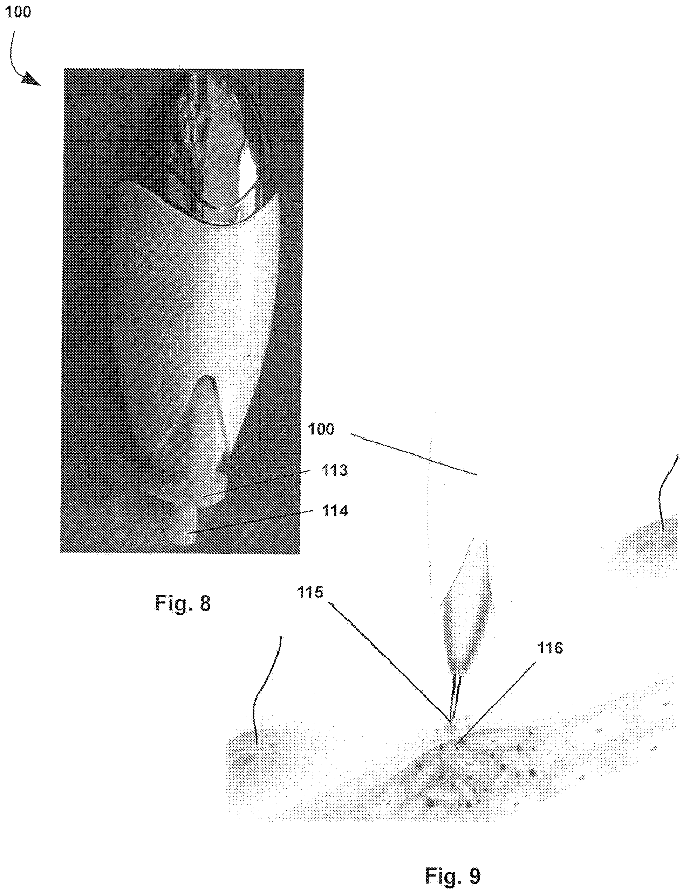

**Figure 9** shows more in detail how tip **115** is placed in touch with a skin **116**, preferably a forearm **122** skin belonging to a person, as that shown in **Figure 4****.**

In order to work, the same electric device **100** further comprises means **50** of generation, transmission and control of an electric pulse, Said means **50** are contained inside said main body **117**, and are electrically connected to the same tip **115.**

The electric device **100** further comprises an outer nozzle **114**, that is made of plastic, having a cylindrical and hollow shape, placed at the top of said main body **117**, that contains said tip **115** inside, so that it is not visible from outside; and including further means with a related tank releasing ink **113.**

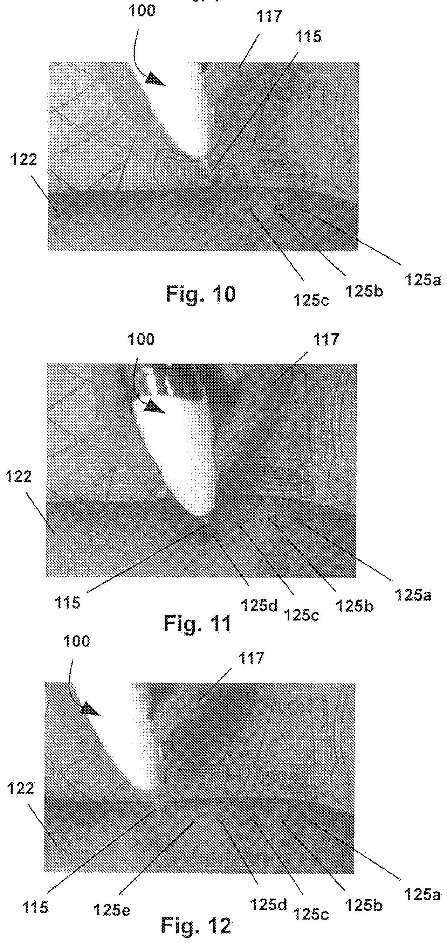

With reference to **Figures 9****,** **10, 11 and 12****,** it is possible to see a sequence of steps related to the use of the device: said nozzle **114** and said tip **115** are placed in touch with a skin **116,** preferably a forearm skin belonging to a person; a series of ink marks, that are shaped as circular areas **125a, 125b,** ..., are printed in sequence on the same skin **116;** an electric pulse is emitted on command, for each of said circular areas **125a, 125b,** ..., so that the same pulse emitted from said tip **115** generates a respective small surface hole on the same skin **116.**

Further according to the invention, the device **100** comprises means for storage of respective substances, by drops or by liquid micro-film, each of said substances being composed of a specific purified allergen.

The tip **301a**, **301b**, ..., of each allergen dispenser is shaped as a small brush, and when it is passed on the skin it releases an allergen micro-film that is smaller than a drop, avoiding therefore the drawback of liquid diffusion and contamination with other points.

With reference to **Figures 1, 2 and 3****,** there are shown means providing on command **203** each of said substances, by drops **202** - e.g. by a pipette or a brush dispenser, from said means for storage to a respective area **201** of said circular areas **125a, 125b,** ... .

In such a way, the drops **202** of substances go in touch with the respective skin **116** surface layers, and it is possible to observe, after a specific time, the occurring of a possible allergy reaction with reference to each of said substances: in case of a positive allergy reaction a so called *hive* **205** shows up, that is a skin edema having a perimetric erythema ring.

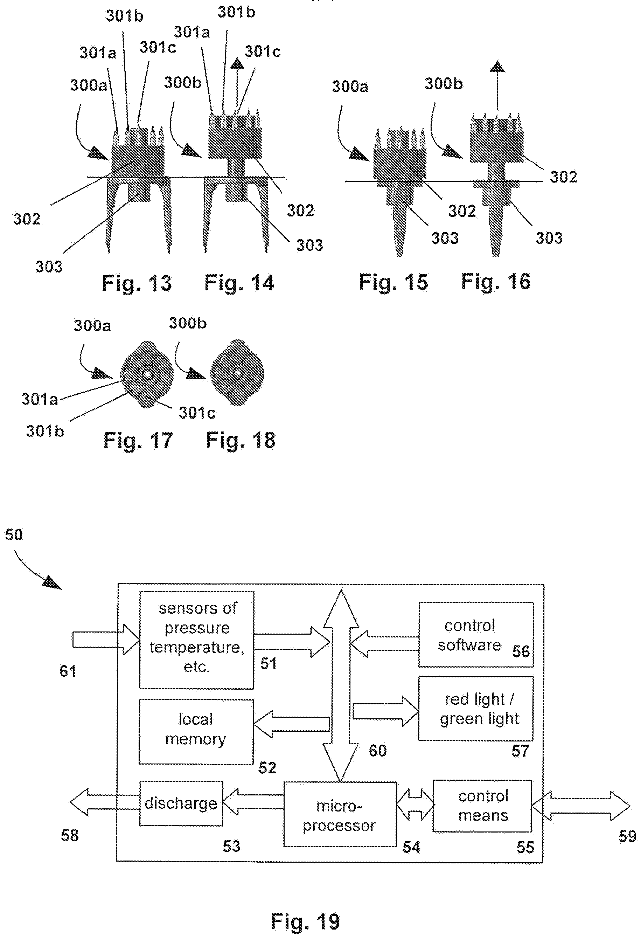

**Figure 19** shows a block diagram of said means **50** of generation, transmission and control of an electric pulse. They are composed of: a micro-processing unit **54;** a data bus **60;** a local memory **52;** a control software **56;** external control means **55,** e.g. represented by buttons; some LED elements **57,** e.g. represented by red light / green light; sensors of external physical magnitudes **51,** e.g. represented by pressure, temperature, conductivity, etc.; and a generator of an electric discharge **53.**

Said means **50** of generation, transmission and control of an electric pulse, can further comprise means tuning on manual command of intensity and duration of the emitted pulse, corresponding to one of the following levels: for more sensitive skins, for less sensitive skins, and for resistant skins (old people).

As an alternative, said means **50** of generation, transmission and control of an electric pulse, can further comprise: sensors for detection of the skin resistance, and means tuning on automatic command (*skin auto-checking*) of intensity and duration of the emitted pulse, corresponding to the type of detected skin.

Said means **50** of generation, transmission and control of an electric pulse, can further comprise means defining the intensity and duration of the emitted pulse according to factory default values.

**Figures 13, 15 and 17** show an add-on element **300a,** in a set back position, applied to the same allergy test device **100.** Said add-on element **300a** comprises a set of elements **301a, 301b**, **301c,** ... , placed on a platform **302** connected to a supporting element **303.**

**Figures 14, 16 and 18** show an add-on element **300b**, in a set front position, applied to the same allergy test device **100.** Said add-on element **300b** comprises a set of elements **301a, 301b, 301c,** ... , placed on a platform **302** connected to a supporting element **303.**

The present invention further discloses a process to achieve a skin allergy test, by using an electric device **100,** as defined according to technical characteristics above described. The process comprises the following steps:
- said nozzle **114** and said tip **115** are placed in touch with a skin **116,** preferably a forearm skin belonging to a person;
- a series of ink marks, that are shaped as circular areas **125a, 125b,** ... , are printed in sequence on the same skin **116;**
- an electric pulse is emitted on command, for each of said circular areas **125a, 125b,** ... , so that the same pulse emitted from said tip **115** generates a small surface hole on the same skin **116;**
- providing on command **203** a specific purified allergen, by drops **202,** to a respective area **201** of said circular areas **125a, 125b,** ... , so that the drops **202** go in touch with the respective skin **116** surface layers;
- observing, after a specific time, the occurring of a possible allergy reaction with reference to each of said allergen substances; in case of a positive allergy reaction a so called *hive* **205** shows up, that is a skin edema having a perimetric erythema ring.

In the above process to achieve a skin allergy test, the intensity and duration of the electric pulse can be defined by: tuning means based on a manual command; or tuning means based on an automatic command (skin *auto-checking*); or means that are set to factory default values.

Therefore, the above examples show that the present invention reaches all the expected objectives. In particular, it permits to achieve electric device for the skin allergy test, to be placed in touch with a skin, preferably a forearm skin belonging to a person; said device is able to print a series of areas by ink marks, and to emit on command an electric pulse, that generates a respective small surface hole on the same skin.

Furthermore, the device comprises means providing on command one or more allergens, by drops - e.g. by a pipette or a brush dispenser, to said areas printed by ink marks, so that the allergens can go in touch with the skin surface layers, and it is possible to observe the occurring of a possible allergy reaction.

Further according to the invention, the intensity and duration of the electric pulse emitted by the device can be defined by: tuning means based on a manual command; or tuning means based on an automatic command (skin *auto-checking*); or means that are set to factory default values.

The invention further comprises an add-on element, in a set back or front position, and that said add-on element comprises a set of elements placed on a platform.

Finally, according to the invention, the same device comprises, at the external surface, a series of buttons for activation and/or selection of the pulse duration and intensity, and it further comprises some LED elements, having green or red color, that indicate different states of working in the same device.

The invention is defined in claim 1. Further aspects and preferred embodiments are defined in the appended claims. Aspects, embodiments and examples of the present disclosure which do not fall under the scope of the appended claims do not form part of the invention and are merely provided for illustrative purposes.

## Claims

1. Electric device (100) for the skin allergy test, comprising:
- a main body (117), where a metallic tip (115) is installed on the top of it;
- means (50) of generation, transmission and control of an electric pulse, said means (50) being contained inside said main body (117), and being electrically connected to the same tip (115);
- an outer nozzle (114), that is made of plastic, having a cylindrical and hollow shape,
placed at the top of said main body (117), that contains said tip (115) inside, so that it is not visible from outside; and including further means with a related tank releasing ink (113),
so that said nozzle (114) and said tip (115) are configured to be placed in touch with a skin (116) belonging to a person; a series of ink marks, that are shaped as circular areas (125a, 125b, ,..), printed in sequence on the same skin (116); an electric pulse configured to be emitted on command, for each of said circular areas (125a, 125b, ...), so that the same pulse emitted from said tip (115) is configured to generate a respective small surface hole on the same skin (116).

2. Electric device (100) for the skin allergy test, according to previous claim 1,
**characterized by** further comprising:
- means for storage of respective substances, each of said substances being composed of a specific purified allergen; and means providing on command (203) each of said substances, by drops (202) - e.g. by a pipette or a brush dispenser, from said means for storage to a respective area (201) of said circular areas (125a, 125b, ...),
so that the drops (202) of substances go in touch with the respective skin (116) surface layers, and it is possible to observe, after a specific time, the occurring of a possible allergy reaction with reference to each of said substances: in case of a positive allergy reaction a so called *hive* (205) shows up, that is a skin edema having a perimetric erythema ring.

3. Electric device (100) for the skin allergy test, according to previous claims 1 or 2,
**characterized in that**:
- said means (50) of generation, transmission and control of an electric pulse, further comprise means tuning on manual command of intensity and duration of the emitted pulse, corresponding to one of the following levels: for more sensitive skins, for less sensitive skins, and for resistant skins.

4. Electric device (100) for the skin allergy test, according to previous claims 1 or 2,
**characterized in that**:
- said means (50) of generation, transmission and control of an electric pulse, further comprise: sensors for detection of the skin resistance, and means tuning on automatic command (*skin auto-checking*) of intensity and duration of the emitted pulse, corresponding to the type of detected skin.

5. Electric device (100) for the skin allergy test, according to one of previous claims from 1 to 4, **characterized in that**:
- said means (50) of generation, transmission and control of an electric pulse, further comprise means defining the intensity and duration of the emitted pulse according to factory default values.

6. Electric device (100) for the skin allergy test, according to one of previous claims from 1 to 5, **characterized in that**:
- said means (50) of generation, transmission and control of an electric pulse, are composed of: a micro-processing unit (54); a data bus (60); a local memory (52); a control software (56); external control means (55), e.g. represented by buttons; some LED elements (57), e.g. represented by red light / green light; sensors of external physical magnitudes (51), e.g. represented by pressure, temperature, conductivity, etc.; and a generator of an electric discharge (53).

7. Electric device (100) for the skin allergy test, according to one of previous claims from 1 to 6, **characterized in that** further comprising:
- an add-on element (300a), in a set back position, applied to the same allergy test device (100); said add-on element (300a) comprises a set of elements (301a, 301b, 301c, ...), placed on a platform (302) connected to a supporting element (303).

8. Electric device (100) for the skin allergy test, according to one of previous claims from 1 to 6, **characterized in that** further comprising:
- an add-on element (300b), in a set front position, applied to the same allergy test device (100); said add-on element (300b) comprises a set of elements (301a, 301b, 301c, ...), placed on a platform (302) connected to a supporting element (303).

## Patentansprüche

1. Elektrisches Gerät (100) für den Hautallergietest, umfassend:
- einen Hauptkörper (117), auf dessen Oberseite eine Metallspitze (115) installiert ist;
- Mittel (50) zur Erzeugung, Übertragung und Steuerung eines elektrischen Impulses, wobei die Mittel (50) innerhalb des Hauptkörpers (117) enthalten und mit derselben Spitze (115) elektrisch verbunden sind;
- eine äußere Düse (114), die aus Kunststoff hergestellt ist und eine zylindrische und hohle Form hat, die an der Oberseite des Hauptkörpers (117) angeordnet ist und die Spitze (115) im Inneren enthält, so dass sie von außen nicht sichtbar ist ; und mit weiteren Mitteln mit einem zugehörigen Tank, der Tinte (113) freisetzt,
so dass die Düse (114) und die Spitze (115) konfiguriert sind, um in Kontakt mit einer Haut (116) gebracht zu werden, die einer Person gehört; eine Reihe von Tintenmarkierungen, die als kreisförmige Bereiche (125a, 125b, ...) geformt sind und nacheinander auf dieselbe Haut (116) gedruckt werden; einen elektrischen Impuls, der so konfiguriert ist, dass er auf Befehl für jeden der kreisförmigen Bereiche (125a, 125b, ...) ausgesendet wird, so dass derselbe Impuls, der von der Spitze (115) ausgesendet wird, so konfiguriert ist, dass er ein entsprechendes kleines Oberflächenloch auf derselben Haut erzeugt (116).

2. Elektrisches Gerät (100) für den Hautallergietest nach dem vorhergehenden Anspruch 1, **dadurch gekennzeichnet, dass** es weiterhin Folgendes umfasst:
- Mittel zur Aufbewahrung jeweiliger Substanzen, wobei jede der Substanzen aus einem spezifischen gereinigten Allergen besteht; und Mittel, die auf Befehl (203) jede der Substanzen tropfenweise (202) - z. durch eine Pipette oder einen Pinselspender, von der Einrichtung zum Aufbewahren zu einem jeweiligen Bereich (201) der kreisförmigen Bereiche (125a, 125b, ...),
so dass die Tropfen (202) von Substanzen mit den jeweiligen Oberflächenschichten der Haut (116) in Kontakt kommen, und es möglich ist, nach einer bestimmten Zeit das Auftreten einer möglichen allergischen Reaktion in Bezug auf jede der Substanzen zu beobachten: in Bei einer positiven Allergiereaktion zeigt sich ein sogenannter Hive (205), also ein Hautödem mit einem perimetrischen Erythemring.

3. Elektrisches Gerät (100) für den Hautallergietest, nach Anspruch 1 oder 2, **dadurch gekennzeichnet, dass**:
- die Mittel (50) zum Erzeugen. Übertragen und Steuern eines elektrischen Impulses ferner Mittel zum manuellen Einstellen der Intensität und Dauer des abgegebenen Impulses entsprechend einem der folgenden Niveaus umfassen: für empfindlichere Haut, für weniger empfindliche Haut, und für widerstandsfähige Häute.

4. Elektrisches Gerät (100) für den Hautallergietest, nach den vorhergehenden Ansprüchen 1 oder 2, **dadurch gekennzeichnet, dass**:
- die Mittel (50) zum Erzeugen, Übertragen und Steuern eines elektrischen Impulses ferner umfassen: Sensoren zum Erfassen des Hautwiderstands und Mittel zum Einstellen auf automatischen Befehl (automatische Hautprüfung) von Intensität und Dauer des emittierten Impulses entsprechend zum erkannten Hauttyp.

5. Elektrisches Gerät (100) für den Hautallergietest, nach einem der vorhergehenden Ansprüche 1 bis 4, **dadurch gekennzeichnet, dass**:
- die Mittel (50) zur Erzeugung, Übertragung und Steuerung eines elektrischen Impulses ferner Mittel umfassen, die die Intensität und Dauer des emittierten Impulses gemäß werkseitig voreingestellten Werten definieren.

6. Elektrisches Gerät (100) für den Hautallergietest, nach einem der vorhergehenden Ansprüche 1 bis 5, **dadurch gekennzeichnet, dass**:
- die Mittel (50) zur Erzeugung, Übertragung und Steuerung eines elektrischen Impulses bestehen aus: einer Mikroprozessoreinheit (54); einen Datenbus (60); einen lokalen Speicher (52); eine Steuersoftware (56); externe Steuermittel (55), z.B. dargestellt durch Schaltflächen; einige LED-Elemente (57), z.B. dargestellt durch rotes Licht / grünes Licht; Sensoren externer physikalischer Größen (51), z.B. dargestellt durch Druck, Temperatur, Leitfähigkeit usw.; und einen Generator einer elektrischen Entladung (53).

7. Elektrisches Gerät (100) für den Hautallergietest, nach einem der vorhergehenden Ansprüche von 1 bis 6, **dadurch gekennzeichnet, dass** es weiterhin umfasst:
- ein Zusatzelement (300a), in einer zurückversetzten Position, angebracht an derselben Allergietestvorrichtung (100); das Zusatzelement (300a) einen Satz von Elementen (301a, 301b, 301c, ...) umfasst, die auf einer Plattform (302) angeordnet sind, die mit einem Stützelement (303) verbunden ist.

8. Elektrisches Gerät (100) für den Hautallergietest, nach einem der vorhergehenden Ansprüche von 1 bis 6, **dadurch gekennzeichnet, dass** es weiterhin umfasst:
- ein Zusatzelement (300b) in einer vordersten Position, angebracht an derselben Allergietestvorrichtung (100); das Zusatzelement (300b) einen Satz von Elementen (301a, 301b, 301c, ...) umfasst, die auf einer Plattform (302) angeordnet sind, die mit einem Stützelement (303) verbunden ist.

## Revendications

1. Appareil électrique (100) pour le test d'allergie cutanée, comprenant :
- un corps principal (117), où une pointe métallique (115) est installée sur le dessus de celui-ci ;
- des moyens (50) de génération, d'émission et de commande d'une impulsion électrique, lesdits moyens (50) étant contenus à l'intérieur dudit corps principal (117), et étant reliés électriquement à la même pointe (115) ;
- une buse extérieure (114), en matière plastique, ayant une forme cylindrique et creuse, placée au sommet dudit corps principal (117), qui contient ladite pointe (115) à l'intérieur, de sorte qu'elle n'est pas visible de l'extérieur ; et comprenant en outre des moyens avec un réservoir associé libérant de l'encre (113),
de sorte que ladite buse (114) et ledit embout (115) sont configurés pour être mis en contact avec une peau (116) appartenant à une personne ; une série de marques d'encre, qui ont la forme de zones circulaires (125a, 125b, ...) imprimées en séquence sur la même peau (116); une impulsion électrique configurée pour être émise sur commande, pour chacune desdites zones circulaires (125a, 125b, ...), de sorte que la même impulsion émise depuis ladite pointe (115) est configurée pour générer un petit trou de surface respectif sur la même peau ( 116).

2. Appareil électrique (100) pour le test d'allergie cutanée, selon la revendication précédente 1, **caractérisé en ce qu'**il comprend en outre :
- des moyens de stockage de substances respectives, chacune desdites substances étant composée d'un allergène purifié spécifique ; et des moyens fournissant sur commande (203) chacune desdites substances, par gouttes (202) - par ex. par une pipette ou un pinceau distributeur, depuis lesdits moyens de stockage jusqu'à une zone respective (201) desdites zones circulaires (125a, 125b, ...),
de sorte que les gouttes (202) de substances entrent en contact avec les couches superficielles de la peau (116) respectives, et qu'il est possible d'observer, après un temps déterminé, la survenue d'une éventuelle réaction allergique en référence à chacune desdites substances : en en cas de réaction allergique positive, une urticaire (205) apparaît, c'est-à-dire un œdème cutané ayant un anneau érythémique périmétrique.

3. Appareil électrique (100) pour le test d'allergie cutanée, selon les revendications précédentes 1 ou 2, **caractérisé en ce que** :
- lesdits moyens (50) de génération, d'émission et de commande d'une impulsion électrique, comprennent en outre des moyens d'accord sur commande manuelle d'intensité et de durée de l'impulsion émise, correspondant à l'un des niveaux suivants : pour les peaux plus sensibles, pour les peaux moins sensibles , et pour les peaux résistantes.

4. Appareil électrique (100) pour le test d'allergie cutanée, selon les revendications précédentes 1 ou 2, **caractérisé en ce que** :
- lesdits moyens (50) de génération, d'émission et de contrôle d'une impulsion électrique, comprennent en outre : des capteurs de détection de la résistance cutanée, et des moyens d'accord sur commande automatique (skin auto-checking) d'intensité et de durée de l'impulsion émise, correspondant au type de peau détecté.

5. Appareil électrique (100) pour le test d'allergie cutanée, selon l'une des revendications précédentes de 1 à 4, **caractérisé en ce que** :
- lesdits moyens (50) de génération, d'émission et de commande d'une impulsion électrique, comprennent en outre des moyens définissant l'intensité et la durée de l'impulsion émise selon des valeurs par défaut d'usine.

6. Appareil électrique (100) pour le test d'allergie cutanée, selon l'une des revendications précédentes de 1 à 5, **caractérisé en ce que** :
- lesdits moyens (50) de génération, d'émission et de commande d'une impulsion électrique, sont composés de : une unité de micro-traitement (54) ; un bus de données (60); une mémoire locale (52); un logiciel de contrôle (56); des moyens de commande externes (55), par ex. représenté par des boutons ; certains éléments LED (57), par ex. représenté par feu rouge / feu vert ; capteurs de grandeurs physiques externes (51), p. représenté par la pression, la température, la conductivité, etc. ; et un générateur d'une décharge électrique (53).

7. Appareil électrique (100) pour le test d'allergie cutanée, selon l'une des revendications précédentes de 1 à 6, **caractérisé en ce qu'**il comprend en outre :
- un élément rapporté (300a), en position reculée, appliqué sur le même dispositif de test d'allergie (100) ; ledit élément rapporté (300a) comprend un ensemble d'éléments (301a, 301b, 301c, ...), placés sur une plate-forme (302) reliée à un élément de support (303).

8. Appareil électrique (100) pour le test d'allergie cutanée, selon l'une des revendications précédentes de 1 à 6, **caractérisé en ce qu'**il comprend en outre :
- un élément rapporté (300b), en position avant fixe, appliqué sur le même dispositif de test d'allergie (100) ; ledit élément rapporté (300b) comprend un ensemble d'éléments (301a, 301b, 301c, ...), placés sur une plate-forme (302) reliée à un élément de support (303).
